# EUROPÄISCHE PATENTSCHRIFT

(11) **EP 1 979 667 B1**
(45) Veröffentlichungstag und Bekanntmachung des Hinweises auf die Patenterteilung: **11.08.2010**
(21) Anmeldenummer: 06830737.0
(22) Anmeldetag: 20.12.2006
(51) Int. Cl.: F16P 1/06, B23K 26/18

(54) **LASERSCHUTZVORRICHTUNG**
LASER PROTECTION APPARATUS
DISPOSITIF DE PROTECTION ANTI-LASER

(30) Priorität: 25.01.2006 DE 102006003450
(43) Veröffentlichungstag der Anmeldung: 15.10.2008
(73) Patentinhaber: Oertel und Troeger AG, 95126 Schwarzenbach/Saale (DE)
(72) Erfinder: OERTEL, Harald, 95361 Ködnitz (DE)
(74) Vertreter: Pröll, Jürgen
(86) Internationale Anmeldenummer: PCT/EP2006/069964
(87) Internationale Veröffentlichungsnummer: WO 2007/085334

(56) Entgegenhaltungen:
- WO-A-94/15557
- WO-A-2004/055833
- DE-A1- 4 113 668
- US-A- 4 638 166
- US-A- 5 151 095
- US-A- 5 225 236
- US-A- 5 309 925
- US-A- 5 992 417

## Beschreibung

Die Erfindung betrifft eine Laserschutzvorrichtung in Form einer versteiften Platte gemäß den Merkmalen des Patentanspruchs 1.

Eine Laserschutzvorrichtung ist beispielsweise aus EP-0 247 797-A1 bekannt. Diese Laserschutzvorrichtung wird als Abdeckung für Patienten bei der Durchführung von Laseroperationen eingesetzt, um den Patienten vor Laserstrahlen zu schützen. Die Schutzwirkung und Festigkeit der vorgenannten Anordnung ist allerdings für diverse Anwendungsfälle nicht ausreichend.

Aus DE-41 13 668-A1 ist eine Laserschutzvorrichtung in Form eines flexiblen Flächengebildes, bestehend aus mindestens einer Vlies- oder Gewebelage und einer Lage aus Aluminiumfolie, die längs in ihren Außenrändern miteinander verbunden ist, offenbart. Nachteilig bei dieser Laserschutzvorrichtung ist jedoch, dass diese flexibel ausgestaltet ist. Es handelt sich um einen flexiblen Materialverbund, welcher keine eigene Steifigkeit und Festigkeit aufweist.

Aus US-A-2004/0022296 ist eine Laserschutzvorrichtung bekannt. Bei dieser Laserschutzvorrichtung sind zwei Lagen vorgesehen, wobei die zweite Lage auf der ersten Lage aufgebracht ist und zur Absorption des Laserstrahls dient. Die zweite Lage besteht aus Metall, vorzugsweise Aluminium.

In DE-C1-196 29 037 wird ein Wandelement einer Schutzvorrichtung vor Laserstrahlen, mit wenigstens einer Innenwand und einer im Wesentlichen parallelen Außenwand, beschrieben. Die Innenwand und die Außenwand bestehen aus Metall, wobei die Innenwand mit einer Beschichtung mit hohem Absorptionsvermögen versehen ist. Die der Innenwand zugewandte Seiteninnenfläche der Außenwand besitzt hingegen nur ein geringes Absorptionsvermögen.

Aus der WO 94/15557 A2 ist eine Vorrichtung zum Schutz vor Laserstrahlen bekannt. Diese Vorrichtung besteht aus einem porösen Silikonschaum. Die Vorrichtung ist als Gardine, Abdeckung oder Band einsetzbar. WO 94/15557 A2 offenbart alle Merkmale des Oberbegriffs des Anspruchs 1.

Aus DE-U1-202 02 061 ist ein Abschirrnbauteil für Laserstrahlen bekannt. Es wird ein lichtstreuendes Material, insbesondere Quarzsand, verwendet. Der Laserstrahl wird von dem Quarzsand aufgeweitet und dadurch seiner gebündelten Energie beraubt.

Ausgehend von diesem Stand der Technik ist es die Aufgabe der vorliegenden Erfindung, eine Vorrichtung aufzuzeigen, welche als Stellwand oder nicht flexible Platte einsetzbar ist.

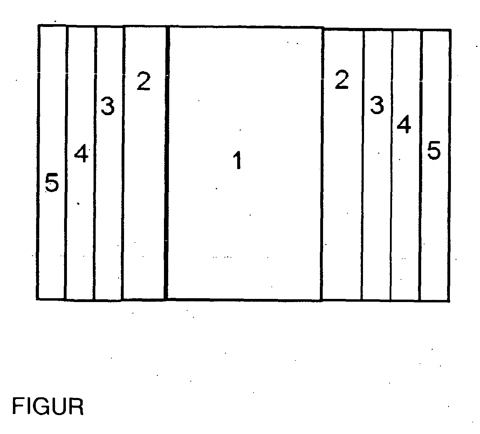

Diese Aufgabe wird durch die Merkmale des Patentanspruchs 1 gelöst. Vorteilhafte Ausgestaltungen der Erfindung ergeben sich anhand der abhängigen Ansprüche und der weiteren Beschreibung sowie der beigefügten Figur.

Die Vorrichtung zum Schutz vor Laserstrahlen besteht aus einem Kern, wobei der Kern aus einer Faserplatte oder aus Schaumstoffplatte, insbesondere in Form eines Hartschaums besteht, und der Kern ein Füllmaterial zur Ableitung von Wärme aufweist, wobei chemische oder thermische Mittel den Kern verfestigen und eine eigensteife Struktur geben, und auf der Oberfläche des Kerns jeweils beidseitig eine erste Schicht unter einer zweiten Schicht angeordnet ist, wobei die erste Schicht eine Schicht zur Formunterstützung des Kerns und eine weitere Schicht zur Absorption eines Laserstrahls aufweist, wobei die zweite Schicht vorzugsweise zum Flammschutz dient. Vorteilhaft hierbei ist, dass der Kern der Vorrichtung auch aus Schaumstoffplatte bestehen kann. Dies macht die Vorrichtung leicht und einfach handhabbar und transportabel. Vorteilhaft ist weiterhin, dass dann der Schaumstoffplatte in Form eines Hartschaums ausgeführt sein kann. Dies erhöht die Festigkeit des Kerns. Vorteilhaft ist außerdem, dass der Kern auch aus einer Faserplatte bestehen kann, wodurch die Dauerelastizität weiter erhöht, sowie die Alterungsbeständigkeit verbessert wird.

Vorteilhaft ist, dass die erste Schicht neben der Formunterstützung des Kerns bereits eine laserabsorbierende Funktion wahrnimmt. Hierdurch wird der Aufbau der Vorrichtung vereinfacht.

Vorteilhaft ist außerdem, dass die zweite Schicht vorzugsweise zur Abführung der in der ersten Schicht bei der Absorption eines Laserstahls entstehenden Wärme/Energie vorgesehen ist und außerdem zum Flammschutz dient. Hierdurch wird neben der Laserabsorption zugleich die Eindämmung einer möglichen Ausbreitung eines Flammenherdes, hervorgerufen durch den Laserstrahl, vorgenommen.

Vorteilhaft gemäß der Ausführung nach Anspruch 2 ist, dass die erste Schicht zur Formunterstützung durch eine Papierschicht, Kartonschicht, Schicht aus Kunststofffolie, Metallfolie oder aus einer Kombination von Papier, Aluminium, Kunststoff ausgestaltet ist. Es werden leicht zu beschaffende Materialien verwendet, was zu einer kostengünstigen Herstellung der Vorrichtung beiträgt.

Vorteilhaft gemäß der Ausführung nach Anspruch 3 ist, dass die laserabsorbierende Funktion der ersten Schicht durch eine Metallfolie oder Aluminiumfolie realisiert ist. Eine Metall- oder Aluminiumfolie dient sowohl als Schutzschicht vor dem Laserstrahl, wie auch zur Stabilisierung des Kerns. Die Ausführung, dass in die zweite Schicht zum Flammschutz Carbonfasern und/oder Fasern aus oxidiertem und thermisch stabilisiertem Polyacrylnitril und/oder Fasern aus Meta-Aramid eingefügt sind, ist ebenfalls vorteilhaft. Dies macht die Vorrichtung flammhemmend.

Gemäß der Ausführung nach Anspruch 4 ist bevorzugt, dass die zweite Schicht eine vliesartige oder textilartige Schicht ist. Dies gewährt eine höhere Biegesteifigkeit, einen besseren Laserschutz und verstärkt die Durchstoßfestigkeit bei mechanischen Verletzungen/Krafteinwirkungen. Außerdem wird eine ansehnliche/ansprechende Optik ermöglicht.

Gemäß der Ausführung nach Anspruch 5 ist bevorzugt, dass die Faserplatte aus Naturfasern, insbesondere Baumwolle, Flachs, Kenaf oder tierischen Fasern, insbesondere Schafwolle, Yakwolle, oder Textilfasern, insbesondere PET, PP, PA, und/oder Mischungen aus Baumwolle und/oder Flachs und/oder Kenaf und/oder Schafwolle und/oder Yakwolle und/oder PET und/oder PP und/oder PA besteht. Durch die Auswahl dieser Materialien wird erreicht, dass die Vorrichtung in Festigkeit, Biegesteifigkeit; Durchstoßfestigkeit modifiziert und angepasst werden kann.

Gemäß der Ausführung nach Anspruch 6 ist bevorzugt, dass die Fasern in der Stärke von 0,7 dtex bis 200 dtex und die Faserlängen in Bereichen von 3 mm bis 150 mm liegen. Dies ermöglicht eine Anpassung an vorgegebene mechanische und akustische Eigenschaften.

Vorteilhaft gemäß der Ausführung nach Anspruch 7 ist, dass in den Kern weitere Füll- und Funktionsstoffe eingebracht sind. Somit wird eine thermische Einwirkung auf eine Stelle des Kerns schnell über den gesamten Kern verteilt. Dies erhöht die Effizienz des wirksamen Laserschutzes.

Vorteilhaft gemäß der Ausführung nach Anspruch 10 ist, dass durch die Verwendung einer Dekorschicht die Vorrichtung im Erscheinungsbild an die Farbgebung des Raumes, in welchem die Vorrichtung eingebracht wird, anpassbar ist. Bei Textilien und insbesondere Folien als Dekorschicht wird die Reinigungsfähigkeit optimiert und ein zusätzlicher Schutz vor mechanischen Beschädigungen erreicht.

Gemäß der Ausführung nach Anspruch 11 ist bevorzugt, dass der Vliesstoffverbund der zweiten Schicht vorzugsweise mit Glas und/oder mit Carbon und/oder mit Fasern aus oxidiertem und thermisch stabilisiertem Polyacrylnitril versetzt ist.

Gemäß der Ausführung nach Anspruch 12 ist bevorzugt, dass aus der Vorrichtung Lamellen oder Platten herstellbar sind. Hierdurch werden Formteile und/oder Planteile geschaffen, welche einfach zu großflächigen Schutzvorrichtungen zusammensetzbar und gut und einfach zu transportieren und montieren sind.

Gemäß der Ausführung nach Anspruch 13 ist bevorzugt, dass die Platten oder Lamellen über eine Nut- und Feder-Konstruktion untereinander verbindbar sind. Dies vereinfacht die Erstellung von großflächigen Schutzvorrichtungen.

Gemäß der Ausführung nach Anspruch 15 ist bevorzugt, dass aus den Lamellen Lamellenvorhänge oder Rollos herstellbar sind. Dadurch ist eine platzsparende Verwahrung bei Nichtgebrauch als auch ein automatisiertes Öffnen/Schließen der Schutzvorrichtung möglich.

Die Vorrichtung besteht aus einem Kern, welcher vorzugsweise aus Hartschaum besteht. Der Hartschaum ist in seiner Ausführung aus Polyurethan bestehend und kann in seiner Konsistenz als hart oder halbhart ausgeführt sein.
Der Kern, der vorzugsweise aus Hartschaum besteht, ist als Platte ausgeführt. Der Kern ist zumindest an einer bzw. an beiden seiner größeren oberflächigen Seiten mit Papier, einer Kunststofffolie, einer Aluminiumfolien-Beschichtung oder einem Vliesstoff beschichtet. Der Vliesstoff enthält vorzugsweise Glas- oder Carbonanteile.

In einer weiteren vorteilhaften Ausgestaltung der Erfindung besteht der Kern aus einer Faserplatte. Diese Faserplatte besteht beispielsweise aus Natunasern wie Baumwolle, Flachs, Kenaf oder tierischen Fasern wie Schafwolle, Yakwolle etc. oder Textilfasern wie z. B. PET, PP, PA, etc. und/oder Mischungen aus diesen Stoffen. Als besonders geeignet haben sich Fasern in der Stärke von 0,7 dtex bis 200 dtex und Faserlängen von 3 mm bis 150 mm ergeben.

Darüber hinaus kann der Kern als Faserplatte, als homogene Mischung aller vorgenannten Komponenten oder als Schichtenaufbau ausgelegt sein. Der Schichtenaufbau erlaubt ebenso die Verwendung von vorgefertigten und im Bedarfsfalle vorverfestigten Fasermatten, auch bekannt als Vliesstoff. Außerdem können textilartige oder textile Stoffe zum Einsatz kommen.
Diese vorverfestigten Vliesstoffe oder textilen Stoffe bestehen vorzugsweise aus den gleichen Fasern, aus denen der Kern, der als Faserplatte ausgeführt ist, besteht. Die

Verfestigung dieser Vliesstoffe kann mechanisch, thermisch, chemisch oder durch eine Kombination aus zwei oder mehreren Verfahren erfolgen.

Zur besseren Wärmeableitung und zur weiteren Erhöhung der Schutzwirkung kann der Kern mit Füllstoffen gefüllt sein.
In vorteilhafter Weise hat sich erwiesen, ein Kalzium-Carbonat als Füllstoff zu verwenoen.
Der Kern aus Hartschaum wird vorzugsweise dadurch hergestellt, dass der Schaum, der als Hartschaum aushärtet, auf eine ebene Fläche aufgebracht wird, vorzugsweise in einer Form, wodurch eine plattenartige Formgebung des Kerns entsteht. Nach dem Aushärten wird der Hartschaum in Form von Platten geschnitten.

Bei Verwendung einer Faserplatte als Kern können zusätzlich zu den Fasern weitere Füll- und Funktionsstoffe in die Faserplatte eingebracht und dort eingebunden werden. Grundsätzlich sind alle Stoffe hierfür geeignet, die sich in eine riesel- und schüttfähige Form bringen lassen. Derartige Füll- und Funktionsstoffe können benutzt werden, um die Beständigkeit im Gebrauch zu verändern bzw. um zusätzliche Eigenschaften in die Faserplatte einzubringen.
Vorzugsweise wird die Faserplatte mittels chemischer oder thermischer Bindemittel verfestigt und erhält so eine eigensteife Struktur. Das Bindemittel selbst kann ebenso ein Füllstoff sein.

Vorzugsweise kann vor dem Zuschnitt bereits auf mindestens eine der Oberflächen der Platte eine Schicht zur Stabilisierung und Verfestigung aufgebracht sein bzw. werden.

Auf den Kern wird zur Erhöhung der Biegefestigkeit, wie auch der Verwindungssteifigkeit und Torsionsspannung, eine erste Schicht, vorzugsweise aus einer Papierlage, einer Kunststofffolie, einer Alufolie oder einer Formkomponente, aufgebracht. Diese erste Schicht trägt vorrangig dazu bei, dem Kern eine höhere Biegesteifigkeit und/oder Verwindungssteifigkeit und/oder Torsionsspannung zu geben.

Hierbei ist insbesondere darauf zu achten, dass diese erste Schicht, welche vorzugsweise auf beide der Großoberflächen des Kerns aufgebracht wird, die Biegesteifigkeit des Kerns und/oder Verwindungssteifigkeit und/oder Torsionsspannung erhöht.

Auf diese erste Schicht wird eine zweite Schicht, vorzugsweise aus laserabsorbierendem Material, aufgebracht, soweit nicht bereits die erste Schicht laserabsorbierend ist. Als vorteilhaft hat sich hierbei die Verwendung einer Metallfolie oder Aluminiumfolie als zweite Schicht ergeben. Diese Folie wird durch thermoplastischen oder duroplastischen Kleber aufgebracht. Es kann aber auch als zweite Schicht ein anderes laserabsorbierendes Material verwendet werden. Über diese zweite Schicht kann eine flammfeste und/oder wärmeabführende dritte Schicht angeordnet werden. Diese optionale dritte Schicht ist insbesondere ein Vlies, vorzugsweise ein Vlies bestehend aus Carbonfasern und/oder Panoxfasem, bzw. ein Vlies, welches mit Carbonfasern und/oder Panoxfasern versetzt ist oder ein textiler Stoff, welcher mit Carbonfasern und/oder Panoxfasern versetzt ist, oder ein textiles Gewebe, welches ebenfalls mit Carbonfasern und/oder Panoxfasern versetzt ist. Unter Panoxfasern werden Fasern aus oxidiertem und thermisch stabilisiertem Polyacrylnitril verstanden. Das Vlies, sprich die dritte Schicht, ist insbesondere in Form eines Vliesverbundstoffes ausgeführt.
Diese dritte Schicht dient zur zusätzlichen Wärmeabführung eines auf die anderen Schichten auftreffenden Laserstahls, welcher vorzugsweise durch die zweite Schicht, der laserabsorbierenden Schicht, in Wärme/Energie umgesetzt wird.

Über der dritten Schicht kann eine Dekorschicht angeordnet werden. Diese Dekorschicht kann aus diversen Materialien bestehen.

In vorteilhafter Weise sind beide großen Oberflächen des Kerns von den genannten Schichten umgeben.

Der Kern in Form einer Schaumstoffplatte kann auch direkt bei der Herstellung auf die erste Schicht aufgeschäumt oder aufgebracht werden. Im Weiteren kann bereits die erste Schicht die Funktion der Laserabsorption und/oder Biegesteifigkeit und/oder Verwindungssteifigkeit und/oder Torsionsspannung übernehmen. Durch diese besondere Ausführungsform wird ein Arbeitsgang bei der Herstellung der Vorrichtung erspart.

Im Weiteren wird die konkrete Ausführung der Erfindung anhand eines Ausführungsbeispieles und anhand einer Figur näher beschrieben.

Die Figur zeigt ein konkretes Ausführungsbeispiel der erfindungsgemäßen Vorrichtung in Form einer Platte im Querschnitt.

Die Figur zeigt einen Kern 1, auf dessen mindestens einer Seite bzw. Oberfläche mindestens eine Schicht angeordnet ist.
In vorteilhafter Weise sind alle großflächigen Oberflächen des Kerns 1 von diesen Schichten umgeben.

Beim Kern 1 handelt es sich um einen Hartschaum, vorzugsweise bestehend aus Polyurethan. Dieser Hartschaum kann in einer harten Ausführung bzw. in einer halbharten Ausführung ausgeführt sein.

In einer weiteren vorteilhaften Ausgestaltung der Erfindung besteht der Kern 1 aus einer Faserplatte. Diese Faserplatte besteht beispielsweise aus Naturfasern wie Baumwolle, Flachs, Kenaf oder tierischen Fasern wie Schafwolle, Yakwolle etc. oder Textilfasern wie z. B. PET, PP, PA, etc. und/oder Mischungen aus diesen Stoffen. Als besonders geeignet haben sich Fasern in der Stärke von 0,7 dtex bis 200 dtex und Faserlängen von 3 mm bis 150 mm ergeben.

Auf dem Kern 1 ist ein- bzw. beidseitig des Kerns 1 eine Schicht 2 zur Stabilisierung angeordnet. Diese Schicht 2 zur Stabilisierung besteht vorzugsweise aus Papier, Karton, aus einem textilen Stoff oder einem textilen Gewebe oder Gewirke oder aus Vliesstoff. Diese Stoffe können zusätzlich vorzugsweise mit Glas- und/oder Carbon- und/oder Panox-Anteilen verstärkt sein. Diese Schicht kann aber auch aus einer metallischen Folie bestehen oder ein Verbundstoff mit metallischem Anteil und/oder ein Vliesstoff oder textiler Stoff oder Gewebe, vorzugsweise mit Glas- oder Carbon-und/oder Panox-Anteil, sein.

Diese Schicht 2 dient vorzugsweise dazu, den Kern 1 in seiner Biegefestigkeit und/oder Verwindungssteifigkeit und/oder Torsionsspannung zu unterstützen. Durch diese Schicht 2 wird vermieden, dass der Kern 1 auf einfache Weise verbogen werden kann.

Über die Schicht 2 ist in vorzugsweiser Ausführung eine weitere Schicht 3 angeordnet. Bei dieser weiteren Schicht 3 handelt es sich um eine laserabsorbierende Schicht. Vorzugsweise kommt hier Aluminiumfolie oder eine andere Metallfolie zum Einsatz.

In einer weiteren Ausführungsform der erfindungsgemäßen Vorrichtung werden die Schicht 2 und die weitere Schicht 3 durch eine einzige Schicht, bestehend aus einem laserabsorbierenden Material, welches zugleich den Kern 1 in seiner Biegefestigkeit und/oder Verwindungssteifigkeit und/oder Torsionsspannung unterstützt, ersetzt. Dieses laserabsorbierende Material ist vorzugsweise in Form einer Metallfolie oder Aluminiumfolie realisiert. Dies hat den Vorteil, dass ein kostengünstiger Aufbau durch Materialersparnis ermöglicht wird, hat jedoch unter Umständen Einfluss auf die Biegefestigkeit der Vorrichtung. Die weitere Schicht 3 dient insbesondere zur Laserabsorption. Wie beschrieben, können aber die Schicht 2 und die weitere Schicht 3 in eine einzige Schicht zusammengefasst werden.

Über der laserabsorbierenden weiteren optionalen Schicht 3 ist eine Schicht 4 angeordnet, welche in vorzugsweiser Ausgestaltung als textiler Stoff oder textiles Gewebe oder Vliesstoff ausgestaltet ist. Diese Schicht 4 dient vorzugsweise zur Abführung der Wärme/Energie, welche durch den auf der weiteren Schicht 3 auftreffenden und dort absorbierten Laserstrahl erzeugt wird.

In vorzugsweiser Ausführung handelt es sich bei Verwendung eines Vliesstoffes um ein Vlies mit Carbon- und/oder Panox-Anteil.

Die Verfestigung des Vliesstoffes erfolgt durch Nadeltechnik, Wasserstrahltechnik, thermische Verfahren oder durch Bindemittel bzw. durch eine Kombination dieser Techniken.

Die Schicht 2, die weitere Schicht 3 und die Vliesstoff-Schicht 4 sind vorzugsweise mit thermo- und duroplastischen Klebersystemen verbunden.

Über dieser Schicht 4 ist eine Dekorschicht 5 angeordnet. Diese Dekorschicht 5 dient dazu, um der Vorrichtung diverse optische Ausgestaltungen zu ermöglichen. Zugleich wird eine verbesserte Reinigungsmöglichkeit erreicht und ein Schutz vor mechanischen Beschädigungen ermöglicht.

In einer vorteilhaften Ausgestaltung der Erfindung ist in den Kern 1 Füllmaterial eingebracht. Bei diesem Füllmaterial handelt es sich in vorzugsweiser Ausführung um Kalzium-Carbonat. Dieses dient zur Abführung gegebenenfalls entstehender Wärme/Energie.

Bei Verwendung einer Faserplatte als Kern 1 werden zusätzlich zu den Fasern weitere Füll- und Funktionsstoffe in die Faserplatte eingebracht und dort eingebunden. Grundsätzlich sind alle Stoffe geeignet, die sich in eine riesel- und schüttfähige Form bringen lassen. Derartige Füll- und Funktionsstoffe werden benutzt, um die Beständigkeit im Gebrauch zu verändern bzw. um zusätzliche Eigenschaften in die Faserplatte einzubringen.
Vorzugsweise wird die Faserplatte mittels chemischer oder thermischer Bindemittel verfestigt und erhält so eine eigensteife Struktur. Das Bindemittel selbst kann ebenso ein Füllstoff sein.

Durch die Vorrichtung wird ein flächiges Element geschaffen, welches eine eigene Stabilität aufweist.

Die Vorrichtung lässt sich in Form von Platten oder Lamellen oder anderen Ausformungen ausgestalten und kann durch eine Nut- und Feder-Kombination untereinander verbunden werden, wodurch sich die einzelnen Platten oder Lamellen zusammenfügen lassen. Durch die Ausgestaltung als Lamellen kann eine Art von Rollo hergestellt werden.

Besonders vorteilhaft bei der erfindungsgemäßen Vorrichtung ist, dass beim Einsatz in Reinräumen keinerlei Verschmutzungen durch die Vorrichtung entstehen und somit die Vorrichtung auch in Reinräumen zum Einsatz kommen kann.

Der Vorteil der Ausgestaltung des Kerns 1 als Faserplatte liegt in der höheren Eigensteifigkeit und der höheren Druckbeständigkeit begründet. Darüber hinaus unterliegt ein Kern 1 als Faserplatte einer deutlich geringeren Alterung und gewährleistet somit einen dauerhafteren Schutz.

Faserplatten als Kern 1 sind vorzugsweise einzusetzen, wenn es um hoch belastende Anwendungen geht, wie z. B. im Maschinenbau.

## Patentansprüche

1. Vorrichtung zum Schutz vor Laserstrahlen, bestehend aus einem Kern (1 ), wobei der Kern (1) aus einer Faserplatte oder aus einer Schaumstoffplatte, insbesondere in Form eines Hartschaums besteht, und der Kern (1) ein Füllmaterial zur Ableitung von Wärme aufweist, wobei chemische oder thermische Mittel den Kern (1) verfestigen und eine eigensteife Struktur geben, **dadurch gekennzeichnet, dass** auf der Oberfläche des Kerns (1) jeweils beidseitig eine erste Schicht (2, 3) unter einer zweiten Schicht (4) angeordnet ist, dass die erste Schicht (2, 3) eine Schicht (2) zur Formunterstützung des Kerns (1) und eine weitere Schicht (3) zur Absorption eines Laserstrahls aufweist und dass die zweite Schicht (4) vorzugsweise zum Flammschutz dient.

2. Vorrichtung nach Anspruch 1,
**dadurch gekennzeichnet, dass**
die Schicht (2) zur Formunterstützung eine Papierschicht, Kartonschicht, Schicht aus Kunststofffolie oder eine Schicht aus Metallfolie oder aus einer Kombination von Papier, Aluminium, Kunststoff besteht.

3. Vorrichtung nach Anspruch 1,
**dadurch gekennzeichnet, dass**
die laserabsorbierende weitere Schicht (3) eine Metallfolie oder Aluminiumfolie ist und/oder in die zweite Schicht (4) zum Flammschutz Carbonfasern und/oder Fasern aus oxidiertem und thermisch stabilisiertem Polyacrylnitril und/oder Fasern aus Meta-Aramid eingefügt sind.

4. Vorrichtung nach Anspruch 1 oder 3,
**dadurch gekennzeichnet, dass**
die zweite Schicht (4) eine vliesartige oder textilartige Schicht ist.

5. Vorrichtung nach Anspruch 1,
**dadurch gekennzeichnet, dass**
die Faserplatte aus Naturfasern, insbesondere Baumwolle, Flachs, Kenaf oder tierischen Fasern, insbesondere Schafwolle, Yakwolle, oder Textilfasern, insbesondere PET, PP, PA, und/oder Mischungen aus Baumwolle und/oder Flachs und/oder Kenaf und/oder Schafwolle und/oder Yakwolle und/oder PET und/oder PP und/oder PA besteht.

6. Vorrichtung nach Anspruch 1 oder 5,
**dadurch gekennzeichnet, dass**
die Fasern in der Stärke von 0,7 dtex bis 200 dtex und die Faserlängen in Bereichen von 3 mm bis 150 mm liegen.

7. Vorrichtung nach einem oder mehreren der Ansprüche 1, 5 oder 6,
**dadurch gekennzeichnet, dass**
zusätzlich weitere Füll- und Funktionsstoffe in den Kern (1) eingebracht sind.

8. Vorrichtung nach einem oder mehreren der Ansprüche 1 oder 7,
**dadurch gekennzeichnet, dass**
das Mittel ein Bindemittel ist und das Bindemittel selbst ein Füllstoff sein kann.

9. Vorrichtung nach Anspruch 7 oder 8,
**dadurch gekennzeichnet, dass**
das Füllmaterial Kalzium-Carbonat ist.

10. Vorrichtung nach einem oder mehreren der vorangehenden Ansprüche, **dadurch gekennzeichnet, dass**
eine dritte Schicht (5) angeordnet ist, welche als Dekorschicht ausgebildet ist.

11. Vorrichtung nach einem oder mehreren der vorangehenden Ansprüche, **dadurch gekennzeichnet, dass**
der Vliesstoffverbund der zweiten Schicht (4) vorzugsweise mit Glas und/oder mit Carbon und/oder mit Fasern aus oxidiertem und thermisch stabilisiertem Polyacrylnitril versetzt ist.

12. Vorrichtung nach einem oder mehreren der vorangehenden Ansprüche, **dadurch gekennzeichnet, dass**
aus der Vorrichtung Lamellen oder Platten herstellbar sind.

13. Vorrichtung nach einem oder mehreren der vorangehenden Ansprüche, **dadurch gekennzeichnet, dass**
die Platten oder Lamellen über eine Nut- und Feder-Konstruktion untereinander verbindbar sind.

14. Vorrichtung nach einem oder mehreren der vorangehenden Ansprüche, **dadurch gekennzeichnet, dass**
die Vorrichtung zum Einsatz in Reinräumen dient.

15. Vorrichtung nach einem oder mehreren der vorangehenden Ansprüche, **dadurch gekennzeichnet, dass**
aus den Lamellen Lamellenvorhänge oder Rollos herstellbar sind.

16. Vorrichtung nach Anspruch 1,
**dadurch gekennzeichnet, dass**
die zweite Schicht (4) zur Abführung der in der laserabsorbierenden weiteren Schicht (3) bei der Absorption eines Laserstahls entstehenden Wärme/Energie dient.

## Claims

1. Device for protection against laser radiation, consisting of a core (1), wherein the core (1) consists of a fibre plate or a foam material plate, particularly in the form of a rigid foam, and the core (1) comprises a filler material for dissipation of heat, wherein chemical or thermal media solidify the core (1) and yield an intrinsically stiff structure, **characterised in that** a respective first layer (2, 3) is arranged under a second layer (4) on the surface of the core (1) on each of two sides, that the first layer (2, 3) comprises a layer (2) for shape support of the core (1) and a further layer (3) for absorption of a laser beam and that the second layer (4) preferably serves for flame protection.

2. Device according to claim 1, **characterised in that** the layer (2) for shape support consists of a paper layer, cardboard layer, layer of plastics material film or a layer of metal foil or of a combination of papers, aluminium and plastics material.

3. Device according to claim 1, **characterised in that** the laser-absorbing further layer (3) is a metal foil or aluminium foil and/or carbon fibres and/or fibres of oxidised and thermally stabilised polyacrylnitrile and/or fibres of meta-aramide are incorporated in the second layer (4) for flame protection.

4. Device according to claim 1 or 3, **characterised in that** a second layer (4) is a non-woven-material-like or textile-like layer.

5. Device according to claim 1, **characterised in that** the fibre plate consists of natural fibres, particularly cotton, flax, gambo fibre or animal fibres, particularly sheep wool or yak wool, or textile fibres, particularly PET, PP or PA, and/or mixtures of cotton and/or flax and/or gambo fibre and/or sheep wool and/or yak wool and/or PET and/or PP and/or PA.

6. Device according to claim 1 or 5, **characterised in that** the fibres lie in thickness from 0.7 dtex to 200 dtex and the fibre lengths in the ranges of 3 mm to 150 mm.

7. Device according to one or more of claims 1, 5 and 6, **characterised in that** in addition further filler and functional materials are incorporated in the core (1).

8. Device according to one or more of claims 1 and 7, **characterised in that** the medium is a binder and the binder itself can be a filler.

9. Device according to claim 7 or 8, **characterised in that** the filler material is calcium carbonate.

10. Device according to one or more of the preceding claims, **characterised in that** a third layer (5), which is formed as a decorative layer, is arranged.

11. Device according to one or more of the preceding claims, **characterised in that** the non-woven material composite of the second layer (4) is preferably mixed with glass and/or with carbon and/or with fibres of oxidised and thermally stabilised polyacrylnitrile.

12. Device according to one or more of the preceding claims, **characterised in that** slats or plates are producible from the device.

13. Device according to one or more of the preceding claims, **characterised in that** the plates or slats are interconnectible by way of groove-and-key construction.

14. Device according to one or more of the preceding claims, **characterised in that** the device serves for use in clean rooms.

15. Device according to one or more of the preceding claims, **characterised in that** slat curtains or roller blinds are producible from the slats.

16. Device according to claim 1, **characterised in that** the second layer (4) serves for conducting away the heat/energy arising in the laser-absorbing further layer (3) on absorption of a layer beam.

## Revendications

1. Dispositif pour protéger des rayons laser, consistant en un noyau (1), le noyau (1) consistant en une plaque en fibres ou en une plaque en matériau expansé, en particulier sous la forme d'une mousse rigide, et le noyau (1) comprenant un matériau de remplissage pour l'évacuation de la chaleur, des moyens chimiques ou thermiques renforçant le noyau (1) et conférant une structure rigide autonome, **caractérisé en ce que**, des deux côtés respectivement, une première couche (2, 3) est disposée sur la surface du noyau (1) sous une deuxième couche (4), **en ce que** la première couche (2, 3) comporte une couche (2) pour le soutien de la forme du noyau (1) et une autre couche (3) pour l'absorption d'un rayon laser et **en ce que** la deuxième couche (4) sert de préférence de protection contre le feu.

2. Dispositif selon la revendication 1,
**caractérisé en ce que**
la couche (2) pour le soutien de forme est une couche de papier, une couche de carton, une couche en un film de plastique ou une couche en une pellicule métallique, ou consiste en une combinaison de papier, d'aluminium et de plastique.

3. Dispositif selon la revendication 1,
**caractérisé en ce que**
l'autre couche (3) absorbant le laser est une pellicule métallique ou une pellicule d'aluminium et/ou **en ce que**, pour la protection pare-feu, des fibres de carbone et/ou des fibres en polyacrylonitrile oxydé et stabilisé thermiquement et/ou des fibres en méta-aramide sont incorporées dans la deuxième couche (4).

4. Dispositif selon la revendication 1 ou 3,
**caractérisé en ce que**
la deuxième couche (4) est une couche de genre non tissé ou de genre textile.

5. Dispositif selon la revendication 1,
**caractérisé en ce que**
la plaque de fibre consiste en fibres naturelles, en particulier en coton, en lin, en kenaf, ou en fibres animales, en particulier en laine de mouton, en laine de yak, ou en fibres textiles, en particulier en PET, en PP ou en PA, et/ou en mélanges de coton et/ou de lin et/ou de kenaf et/ou de laine de mouton et/ou de laine de yak et/ou de PET et/ou de PP et/ou de PA.

6. Dispositif selon la revendication 1 ou 5,
**caractérisé en ce que**
les fibres existent en une épaisseur de 0,7 à 200 dtex et les longueurs des fibres sont comprises dans l'intervalle de 3 mm à 150 mm.

7. Dispositif selon l'une quelconque ou plusieurs des revendications 1, 5 ou 6, **caractérisé en ce que**
d'autres produits de remplissage et d'autres produits fonctionnels sont incorporés en plus dans le noyau (1).

8. Dispositif selon l'une quelconque ou plusieurs des revendications 1 ou 7, **caractérisé en ce que**
le moyen est un liant et **en ce que** le liant peut être lui-même un produit de remplissage.

9. Dispositif selon la revendication 7 ou 8,
**caractérisé en ce que**
le matériau de remplissage est du carbonate de calcium.

10. Dispositif selon l'une quelconque ou plusieurs des revendications précédentes,
**caractérisé en ce que**
une troisième couche (5) est disposée, laquelle est configurée comme couche décorative.

11. Dispositif selon l'une quelconque ou plusieurs des revendications précédentes,
**caractérisé en ce que**
le composite de non tissé de la deuxième couche (4) est additionné de préférence de verre et/ou de fibres de carbone et/ou de fibres en polyacrylonitrile oxydé et stabilisé thermiquement.

12. Dispositif selon l'une quelconque ou plusieurs des revendications précédentes,
**caractérisé en ce que**
partant du dispositif, on peut fabriquer des lamelles ou des plaques.

13. Dispositif selon l'une quelconque ou plusieurs des revendications précédentes,
**caractérisé en ce que**
les lamelles ou les plaques peuvent être assemblées au moyen d'une construction à tenon et mortaise.

14. Dispositif selon l'une quelconque ou plusieurs des revendications précédentes,
**caractérisé en ce que**
le dispositif sert à une utilisation dans des salles blanches.

15. Dispositif selon l'une quelconque ou plusieurs des revendications précédentes,
**caractérisé en ce que**
en partant des lamelles, on peut fabriquer des rideaux de lamelles ou des stores.

16. Dispositif selon la revendication 1,
**caractérisé en ce que**
la deuxième couche (4) sert à l'évacuation de la chaleur/de l'énergie apparaissant dans la couche supplémentaire (3) absorbant le laser lors de l'absorption d'un laser.
